(19) Europäisches Patentamt
European Patent Office
Office européen des brevets

(11) **EP 4 764 506 A1**

(12) **EUROPEAN PATENT APPLICATION**
published in accordance with Art. 153(4) EPC

(43) Date of publication:
**24.06.2026 Bulletin 2026/26**

(21) Application number: **24854220.1**

(22) Date of filing: **14.08.2024**

(51) International Patent Classification (IPC):
*G01N 35/08* (2006.01)     *B01J 19/00* (2006.01)
*B81B 1/00* (2006.01)     *B81C 1/00* (2006.01)
*G01N 37/00* (2006.01)

(52) Cooperative Patent Classification (CPC):
**B01J 19/00; B81B 1/00; B81C 1/00; G01N 35/08;
G01N 37/00**

(86) International application number:
**PCT/JP2024/029002**

(87) International publication number:
**WO 2025/037631 (20.02.2025 Gazette 2025/08)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **16.08.2023 JP 2023132576**

(71) Applicant: **ZACROS Corporation
Tokyo 112-0002 (JP)**

(72) Inventors:
• **ABE, Masato
Tokyo 112-0002 (JP)**
• **HOSOKAWA, Kazuya
Tokyo 112-0002 (JP)**

(74) Representative: **Watermeyer, Nicholas
Withers & Rogers LLP
2 London Bridge
London SE1 9RA (GB)**

(54) **MICROCHIP FOR ANALYZING LIQUID SAMPLE**

(57)     A microchip for liquid sample analysis having a flow path and a liquid reservoir therein, comprising: a substrate having a bonding surface provided with a groove forming the flow path, a depression provided in a part of the groove and serving as a liquid reservoir storing a liquid, and a wall part provided between the groove and the depression to block the outflow of the liquid from the liquid reservoir to the flow path; and
a film bonded to the bonding surface of the substrate, covering the groove to form the flow path and the liquid reservoir, wherein
among the bonded areas of the substrate and the film, the bonded portion between an end face of the wall part of the substrate and the film has a first bonding strength such that it peels off upon application of pressure of 30 kPa or more and less than 100 kPa, and the other bonded areas have a bonding strength stronger than the first bonding strength.

FIG. 1

**Description**

Technical Field

The present invention relates to a microchip for liquid sample analysis.

Background Art

It is known to analyze components in a liquid sample, such as blood, by introducing the liquid sample into a flow path within a microchip and reacting it with a reaction solution containing antibodies or the like at a reaction site provided in the middle of the flow path. As a method for producing such a microchip, a method is known in which a substrate having a groove serving as a flow path formed on its surface is bonded to a film or another substrate using an adhesive.

Patent Document 1 discloses a microchip in which a reagent to be reacted with a liquid sample is stored in a recess in advance and sealed with a film. However, this microchip is used by peeling off the film before use, and it is not intended for use by passing liquid through while the liquid remains sealed within the film. Therefore, there has been a demand for a method to more inexpensively and simply produce a microchip that allows liquid, such as a reagent stored inside, to be easily passed into a flow path with a simple operation.

Prior Art Documents

Patent Documents

Patent Document 1: JP 2009-168667A

Summary of Invention

Problem to be Solved by the Invention

An object of the present invention is to provide a microchip, formed by bonding a substrate and a film and having a flow path and a liquid reservoir therein, which allows the liquid stored inside to be easily passed into the flow path by applying pressure at the time of use.

Means for Solving the Problem

The present inventors conducted extensive studies to solve the above problem. As a result, they found that in a microchip for liquid sample analysis having a flow path and a liquid reservoir therein, which is formed by bonding: a substrate having a bonding surface provided with a groove forming the flow path, a depression provided in a part of the groove and serving as a liquid reservoir storing a liquid, and a wall part provided between the groove and the depression to block the outflow of the liquid from the liquid reservoir to the flow path; and a film bonded to the bonding surface of the substrate, covering the groove to form the flow path and the liquid reservoir; wherein, among the bonded areas of the substrate and the film, the bonded portion between the end face of the wall part of the substrate and the film has a first bonding strength such that it peels off upon application of pressure of 30 kPa or more and less than 300 kPa, and the other bonded areas have a bonding strength stronger than the first bonding strength, a microchip that stably retains liquid can be easily manufactured, and the obtained microchip allows the liquid stored inside to be easily passed into the flow path at the time of use by applying pressure to peel the bond between the end face of the wall part and the film.

That is, the microchip of the present invention is:

A microchip for liquid sample analysis having a flow path and a liquid reservoir therein, comprising: a substrate having a bonding surface provided with a groove forming the flow path, a depression provided in a part of the groove and serving as the liquid reservoir storing a liquid, and a wall part provided between the groove and the depression to block outflow of the liquid from the liquid reservoir to the flow path; and a film bonded to the bonding surface of the substrate, covering the groove to form the flow path and the liquid reservoir, wherein among the bonded areas of the substrate and the film, the bonded portion between the end face of the wall part of the substrate and the film has a first bonding strength such that it peels off upon application of pressure of 30 kPa or more and less than 300 kPa, and the other bonded areas have a bonding strength stronger than the first bonding strength.

In one embodiment, said microchip, wherein

the bonded portion between the end face of the wall part of the substrate and the film has been bonded with an adhesive having a gel fraction after curing of 60% or more, and

for the other bonded areas of the substrate and the film, the bonding surface of the substrate has been surface-treated to have a water contact angle of 10° to 40° and bonded to the film with an adhesive having a gel fraction after curing of 60% or more, can be provided.

In another embodiment, said microchip, wherein

the bonded portion between the end face of the wall part of the substrate and the film has been bonded with an adhesive having a gel fraction after curing of 60% or more, and

the other bonded areas have been joined by welding, the microchip can be provided.

In another embodiment, said microchip, wherein

the end face of the wall part has been surface-treated to have a water contact angle of 110° or more and the bonded portion between the end face of the wall part of the substrate and the film has been bonded with an adhesive having a gel fraction after curing of 60% or more, and

the other bonded areas have been bonded with an adhesive having a gel fraction after curing of 60% or more, the microchip can be provided.

In another embodiment, said microchip, wherein

the bonded portion between the end face of the wall part of the substrate and the film has been surface-treated such that the end face of the wall part has a water contact angle of 110° or more, or

the bonding areas of the substrate other than the end face of the wall part have been surface-treated to have a water contact angle of 10° to 40°, and then

the end face of the wall part of the substrate and the film have been bonded with an adhesive having a gel fraction after curing of 60% or more applied only in a region of the end face covering 1/2 or less of the end face on the downstream side of the flow path, and the other bonded areas have been bonded with an adhesive having a gel fraction after curing of 60% or more, can be provided.

Effects of the Invention

According to the present invention, provide is a microchip that can stably retain liquid during storage and allows the liquid to be easily passed into a flow path by applying pressure at the time of use.

Brief Description of Drawings

[FIG. 1] FIG. 1 is a diagram showing the substrate of the microchip before applying an adhesive.
[FIG. 2] FIG. 2 is a diagram showing the substrate of the microchip after applying an adhesive.
[FIG. 3] FIG. 3 is a completed view of the microchip with a film bonded to the substrate.
[FIG. 4] FIG. 4 is a diagram showing a part of the A-A cross-section of the microchip.
[FIG. 5] FIG. 5 is a diagram showing the microchip in a state where the adhesive has peeled and liquid has passed into the liquid reservoir.
[FIG. 6] FIG. 6 is a diagram showing a part of the B-B cross-section of the microchip after liquid has passed into the liquid reservoir.
[FIG. 7] FIG. 7 is a diagram showing the microchip in a state where the adhesive has peeled and liquid has passed from the liquid reservoir to the downstream flow path.
[FIG. 8] FIG. 8 is a diagram showing a part of the B-B cross-section of the microchip after liquid has passed from the liquid reservoir to the downstream flow path.
[FIG. 9] FIG. 9 is a diagram showing the structure of the substrate of the microchips of Examples 1, 2, and 3.

Description of Embodiments

The present invention relates to:

a microchip for liquid sample analysis having a flow path and a liquid reservoir therein,
comprising: a substrate having a bonding surface provided with a groove forming the flow path, a depression provided in a part of the groove and serving as the liquid reservoir storing a liquid, and a wall part provided between the groove and the depression to block outflow of the liquid from the liquid reservoir to the flow path; and a film bonded to the bonding surface of the substrate, covering the groove to form the flow path and the liquid reservoir, wherein among the bonded areas of the substrate and the film, the bonded portion between the end face of the wall part of the substrate and the film has a first bonding strength such that it peels off upon application of pressure of 30 kPa or more and less than 300 kPa, and the other bonded areas have a bonding strength stronger than the first bonding strength.

When the microchip is used for analyzing a specimen such as a biological sample, the liquid stored in the liquid reservoir is preferably a solution or suspension or washing liquid of a reagent (reactive substance) that reacts with an analyte contained in the sample to be analyzed. Here, reactions include biological reactions, chemical reactions, etc., and biological reactions also include binding reactions. Examples of reagents include, but are not limited to, proteins (including peptides) such as antibodies, enzymes, and receptors, nucleic acids such as DNA and RNA, sugar chains, and low-molecular-weight compounds that bind to biological components. These substances may be labeled with a fluorescent substance or the like, or may be immobilized on a carrier or the like. For example, substances such as antibodies that specifically bind to a target (detection target) substance in a specimen, enzyme proteins that use the target substance as a substrate, blood coagulation factors such as PT reagents, and the like can be mentioned. When the target substance is a nucleic acid, the reactive substance may be a nucleic acid probe, a polymerase (nucleic acid amplification enzyme) for amplifying the nucleic acid, or the like.

In addition, when the microchip is used for culturing or analyzing cells or microorganisms, the liquid stored in the liquid reservoir may be a medium for cells or microorganisms.

In a part of the groove forming the flow path of the microchip substrate, a depression is formed which serves as a liquid reservoir, a region for storing liquid, and the liquid is stored in this depression serves as a liquid reservoir, in advance.

Then, with the liquid stored in the depression, the film is bonded, thereby forming the flow path and the liquid reservoir.

A wall part is provided between the liquid reservoir and the flow path, preferably at the connection part between the liquid reservoir and the flow path, and more preferably at the connection part between the liquid reservoir and the upstream flow path and the connection part between the liquid reservoir and the downstream flow path, to block the outflow of liquid from the liquid reservoir to the flow path. The end face of the wall part is bonded to the film, whereby the liquid is retained within the liquid reservoir and sealed so as not to flow out into the flow path. If either the upstream flow path or the downstream flow path does not exist, the wall part only needs to be at the connection part between the liquid reservoir and the upstream flow path or the downstream flow path.

The bonded portion between the end face of the wall part of the substrate and the film has been bonded with a first bonding strength such that it peels off upon application of pressure of 30 kPa or more and less than 300 kPa. Therefore, by applying a pressure of 30 kPa or more and less than 300 kPa into the microchip, the end face of the wall part and the film peel apart, and the liquid is passed from the liquid reservoir into the flow path.

On the other hand, among the bonding surfaces of the substrate, the bonded areas other than the end face of the wall part (the regions excluding the end face of the wall part, the groove serving as the flow path, the depression serving as the liquid reservoir, and, if present, the through-hole serving as the inlet, the through-hole serving as the outlet, and the groove serving as the waste liquid reservoir) have been bonded to the film with a bonding strength stronger than the first bonding strength. Therefore, only the bonded part between the end face of the wall part and the film can be selectively peeled, allowing the liquid passage to be controlled.

If the pressure for peeling the end face of the wall part and the film is less than 30 kPa, the risk of unexpected peeling due to operations not intended for liquid passage or slight impacts increases. If it is 300 kPa or more, controlling the liquid immediately after passage becomes difficult. Furthermore, at 300 kPa or more, the risk of breakage in unexpected areas other than the end face of the wall part also increases, making it unsuitable for practical use of the microchip. For this reason, the pressure for peeling the end face of the wall part and the film is preferably 30 kPa or more and less than 300 kPa.

Hereinafter, the microchip for liquid sample analysis of the present invention and its manufacturing method will be described with reference to the drawings. However, the following is merely an example, and the manufacturing method of the present invention and the microchip obtained thereby are not limited to the following embodiments.

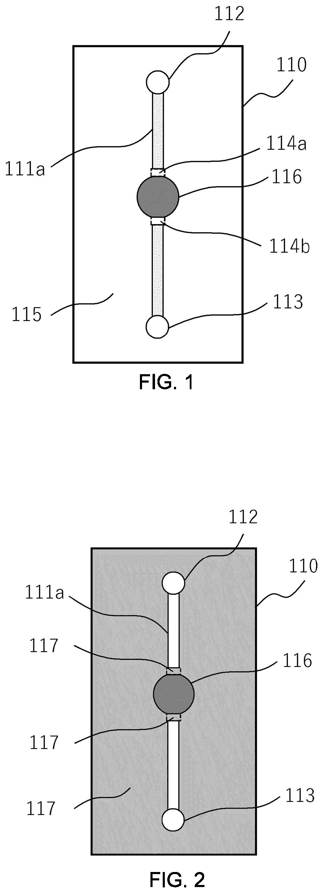

FIG. 1 shows a plan view of a substrate 110 on the surface of which a groove 111a forming a flow path 111 of a microchip 100 is carved. In a part of the groove 111a, a depression is provided which serves as a liquid reservoir 116, a region for storing liquid. Between the liquid reservoir 116 and the groove 111a, wall parts 114a and 114b are provided on the upstream and downstream sides, respectively, to block the outflow of liquid. The surfaces of the wall parts 114a and 114b facing the film 120 to be bonded are also referred to as (wall part) end faces 114a and 114b. The surface on which the groove 111a is carved is the surface to be bonded with the film 120, and is also referred to as a bonding surface 115.

At one end of the groove 111a upstream of the liquid reservoir 116, a through-hole serving as an inlet 112 for pressurizing the liquid is provided, and at the other end, a through-hole serving as an outlet 113 (discharge port) for the liquid or air to flow out is provided. The liquid stored in the liquid reservoir 116 is pressurized using, for example, a microsyringe or a pump. Pressurization of the liquid can also be performed by pressing with a finger or a simple pressurizing member that seals the inlet 112.

Two or more flow paths 111 may be provided. The shape of the flow path 111 is not limited and may be linear or curved. Further, the flow path 111 may have branches. In that case, two or more inlets 112, liquid reservoirs 116, wall parts 114a and 114b, and/or outlets 113 may be present.

For example, a liquid sample is stored in a first liquid reservoir provided in a first flow path, and a reaction substrate liquid (reactive substance) is stored in a second liquid reservoir provided in a second flow path. The liquid sample and the reaction substrate liquid are pressurized from their corresponding inlets (pressurizing ports), whereby the end faces of the wall parts and the film peel apart, and the liquids are passed into the flow paths beyond the liquid reservoirs. The first flow path and the second flow path merge beyond the first liquid reservoir and the second liquid reservoir. By providing a reaction site in the merging region, the liquid sample reacts with the reaction substrate liquid. In this case, the outlet may be provided at the downstream end of the merged flow path.

Alternatively, a washing liquid may be stored in a third liquid reservoir provided in a third flow path, and the washing liquid may be passed into the flow path of the microchip 100 after the reaction between the liquid sample and the reaction substrate liquid. For example, the third flow path is formed to merge upstream of the first liquid reservoir and the second liquid reservoir, and the washing liquid is passed into the first flow path and the second flow path by pressurization from an inlet (pressurizing port).

The inlet 112 and the outlet 113 may be provided on either the substrate 110 side or the film 120 side. For example, the groove 111a serving as the flow path 111 may be provided on the substrate 110, and a film 120 having through-holes at positions overlapping both ends of the groove 111a may be prepared and bonded to the substrate 110. Also, one of the through-holes serving as the inlet 112 and the outlet 113 may be provided in the substrate 110, and the other in the film 120.

The cross-sectional shape of the groove 111a serving as the flow path 111 is arbitrary, such as concave, U-shaped, or V-shaped. The depth of the groove 111a serving as the flow path 111 is preferably 10 to 1000 $\mu$m. The width of the groove 111a is preferably 10 $\mu$m to 4 mm, and particularly preferably 50 $\mu$m to 4 mm. If the flow path width is less than 10 $\mu$m, the risk of blockage due to adhesive flowing into the flow path increases, and with welding technology, bonding is not possible because the flow path shape deforms due to high heat. Bonding with welding technology is difficult at 10 $\mu$m or more and less than 50 $\mu$m, but bonding can be achieved using an adhesive. At 50 $\mu$m to 2 mm, good bonding is attained with either method. On the other hand, if the width exceeds 4 mm, the possibility of causing unexpected rupture at locations other than the end face 114a of the wall part 114 when pressure is applied to penetrate the wall part 114 increases. This is because as the area of the film 120 not bonded to the substrate 110 increases, the peeling force applied to the unbonded film surface increases when pressure is applied within the flow path to penetrate the end face of the wall part. The length of the portion corresponding to the flow path 111, including the thickness (length in the longitudinal direction of the flow path 111) of the wall part 114, is, for example, 3 mm to 5 cm. The width of the groove 111a may be constant or may vary. The depth of the groove 111a may also be constant or may vary.

To control the direction of liquid flow, the groove 111a serving as the flow path 111 of the substrate 110, the part of the film 120 covering the flow path 111, the wall surfaces on both sides of the wall part 114, etc., are hydrophilized. The hydrophilic treatment may also be applied to the depression serving as the liquid reservoir 116 and the part of the film 120 covering the liquid reservoir 116.

The width of the groove 111a (width of the flow path 111) may be made narrower than the width of the wall parts 114a and 114b. By making the width of the wall parts 114a and 114b wider than the width of the flow path 111, the flow rate of the liquid passing through the gap between the end faces 114a and 114b of the wall parts 114a and 114b and the film 120 increases, making it easier for the liquid to flow.

The depression serving as the liquid reservoir 116 may be of any size sufficient to store a desired amount of liquid, and its shape is not particularly limited. For example, it may be cylindrical or prismatic, and by increasing the area and depth, more liquid can be stored. The area of the depression is, for example, 0.1 to 50 mm$^2$, and in the case of a circular liquid reservoir 116, its diameter is, for example, 0.2 to 6 mm. However, the area may change according to the depth of the groove, and for example, it may be a mortar-shaped depression. The depth of the depression is preferably deeper than the depth of the groove serving as the flow path, for example, 20 $\mu$m to 3 mm. If the area of the depression, i.e., the area of the film 120 not bonded to the substrate 110, becomes large, there is a risk that the liquid reservoir will rupture at a pressure lower than that at which the wall part 114 is penetrated. To prevent unexpected rupture of the liquid reservoir, for example, when the area of the depression exceeds 4 mm$^2$, it is desirable that the bonded portion between the end face of the wall part of the substrate 110 and the film 120 is bonded with an adhesive having a gel fraction after curing of 60% or more, applied only in a region of the end face covering 1/2 or less of the end face on the downstream side of the flow path, after the end face of the wall part has been surface-treated to have a water contact angle of 110° or more, or the bonding part of the substrate other than the end face of the wall part has been surface-treated to have a water contact angle of 10° to 40°. By this method, only the end face of the wall part can be penetrated at a relatively low pressure of 30 to 80 kPa, and unexpected rupture of the liquid reservoir can be prevented. To prevent rupture of the liquid reservoir, the deflection of the film can also be physically suppressed when pressure is applied to the liquid reservoir by bonding a plate material with a thickness of 0.5 mm or more only to the location of the liquid reservoir on the film surface. As a means to physically suppress the deflection of the film, even without bonding a plate material to the location of the liquid reservoir on the film surface, mechanically pressing the same location can also prevent the rupture of the liquid reservoir.

The wall parts 114a and 114b are provided to block the liquid stored in the liquid reservoir 116 from flowing out into the flow path 111.

The thickness (length in the longitudinal direction of the flow path 111) of the wall parts 114a and 114b may be any thickness that allows the pressurized liquid to peel the end faces 114a and 114b of the wall parts 114a and 114b from the film 120, allowing the liquid to pass from the liquid reservoir 116 to the flow path 111 side, but it is preferably 400 $\mu$m to 5 mm. Even when the thickness of the adhesive film applied to the substrate 110 is a thin film of 5 to 10 $\mu$m, when the film 120 is pressure-bonded, the edge of the applied adhesive spreads by about 100 to 200 $\mu$m, depending on the pressure. Therefore, if the length of the wall part is less than 400 $\mu$m, the embodiment in which the adhesive is applied only to a region of half or less of the end face of the wall part becomes difficult. On the other hand, if the length of the wall part exceeds 5 mm, the amount of liquid passing through the end face of the wall part after penetration is small, which is not suitable for practical use. The thickness of the wall parts 114a and 114b may be constant or may vary. The wall surfaces of the wall parts 114a and 114b may be inclined surfaces such that the thickness of the wall parts 114a and 114b becomes thinner toward the end faces 114a and 114b. By making the wall surfaces of the wall parts 114a and 114b inclined or stepped, and constructing the structure such that the depth of the liquid reservoir 116 becomes shallower closer to the wall parts 114a and 114b, the liquid easily flows between the end faces 114a and 114b and the film 120, and the end faces 114a and 114b and the film 120 are more easily peeled. Specifically, by making the location 1 mm to 4 mm from the wall parts 114a and 114b in the direction of the pressure source shallower, it can be easier to peel the wall parts 114a and 114b and the film 120. If the distance from the wall part to the shallow location is less than 1 mm, the area where the film 120 deflects when pressure is applied is small, making it difficult to obtain the effect of facilitating peeling. On the other hand, if it exceeds 4 mm, the area where the film 120 deflects when pressure is applied is large, making it difficult to penetrate only the wall part. The depth should be shallower than the groove 111, but is more preferably 5 to 30 $\mu$m. The height of the wall parts 114a and 114b may be formed at substantially the same height as the bonding surface 115, or may be formed lower than the bonding surface 115.

The size of the through-hole serving as the inlet 112 may be any size that allows the liquid in the liquid reservoir 116 to be pressurized using a microsyringe or a pump connected via an adapter. The size of the through-hole serving as the inlet 112 is, for example, 0.2 to 3 mm in diameter. The size of the through-hole serving as the outlet 113 is not particularly limited as long as it functions as an outlet for the liquid sample. The size of the through-hole serving as the outlet 113 is, for example, 0.2 to 3 mm in diameter.

The material of the microchip 100 (substrate) can be metal, glass, plastic, silicone, or the like, but from the viewpoint of detecting reactions by luminescence, color development, or visually, a transparent material is preferable, and transparent plastic is more preferable. Examples of the material of the microchip 100 include polyethylene, polypropylene, polystyrene, polymethyl methacrylate, cycloolefin polymer, cycloolefin copolymer, polyphenylene oxide, polyethylene terephthalate, polyethylene naphthalate, polycarbonate, polyamide, polyimide, phenol resin, epoxy resin, polyvinylidene chloride, polyvinyl chloride, acrylonitrile-butadiene-styrene (ABS) resin, poly(2-methoxyethyl acrylate) (PMEA) resin, and the like.

The groove 111a and through-holes provided in the substrate 110 of the microchip 100 can be carved with a blade or a laser beam, but when the material of the microchip 100 is plastic, they can also be formed by injection molding. Forming by injection molding is preferable because the microchip 100 can be efficiently produced with consistent quality.

The material of the film 120 is preferably transparent plastic, and more preferably polyethylene terephthalate (PET) resin, cycloolefin polymer (COP) resin, cycloolefin copolymer (COC) resin, polystyrene (PS) resin, polycarbonate (PC) resin, or polymethyl methacrylate (PMMA) resin.

In addition, it is preferable to use a moisture-proof film as the film, as this can prevent the evaporation of the stored liquid. The degree of moisture-proofness is, for example, a water vapor transmission rate of 1.5 g/m$^2$·day or less, preferably 0.25 to 1.5 g/m$^2$·day, and more preferably 0.25 to 0.5 g/m$^2$·day. The moisture-proof film may be polytetrafluoroethylene (PCTFE) alone, a multilayer film containing PCTFE, or a vapor-deposited film. Examples of multilayer films containing PCTFE include a 4-layer film consisting of PCTFE, maleic anhydride modified polyethylene (MAPE), linear low-density polyethylene (LL), and cycloolefin polymer (COP) resin from the outermost layer, and a 5-layer film consisting of PCTFE, MAPE, ethylene vinyl alcohol copolymer resin (EVOH), MAPE, and COP from the outermost layer. To obtain moisture-proofness, the thickness of the PCTFE layer is preferably 10 $\mu$m or more, and more preferably 20 $\mu$m.

Examples of vapor-deposited films include films of polyethylene terephthalate (PET) vapor-deposited with aluminum, alumina, or silica, and films of nylon vapor-deposited with aluminum, alumina, or silica.

Aluminum may be used alone.

The moisture-proof film is not limited to the above, and may be, for example, a multilayer film containing one or more types of films such as polytetrafluoroethylene (PTFE) and perfluoroalkoxyalkane (PFE).

The total thickness of the film 120 is, for example, preferably 10 to 200 μm, and more preferably 100 to 200 μm.

The Young's modulus (tensile elastic modulus) of the film material is, for example, preferably 50 to 2800 MPa, and more preferably 70 to 1000 MPa. By using a film with the above total thickness and Young's modulus, it becomes possible to specifically peel only the wall part end face 114a by applying pressure. Although it depends on the film material, if the total thickness of the film 120 exceeds 200 μm and the Young's modulus of the film exceeds 2800 MPa, the film does not deform even when pressure is applied, making it difficult to penetrate the wall part 114.

The microchip 100 is obtained by bonding the substrate 110 and the film 120 with the liquid stored in the liquid reservoir 116 of the substrate 110.

Among the bonded areas of the substrate 110 and the film 120, the bonded part between the end faces 114a and 114b of the wall part of the substrate 110 and the film 120 has a first bonding strength such that it peels off upon application of pressure of 30 kPa or more and less than 300 kPa, and the other bonded areas have a bonding strength stronger than the first bonding strength. The bonding strength stronger than the first bonding strength is not particularly limited as long as it is stronger than the first bonding strength, but it is, for example, a bonding strength that does not peel upon application of pressure of at least 300 kPa.

That is, a first bonding treatment is applied to the bonded portion between the end face of the wall part of the substrate and the film, and a second bonding treatment is applied to the other bonded areas.

Here, "the other bonded areas" means the bonded areas other than the bonded portion between the end face of the wall part of the substrate and the film, among the bonded areas of the substrate and the film. Specifically, it means the bonded areas between the film and the regions on the bonding surface of the substrate excluding the groove serving as the flow path, the depression serving as the liquid reservoir, the through-holes serving as the inlet and outlet, and the above-mentioned end face of the wall part (hereinafter also referred to as the bonding region other than the end face of the wall part). If a depression serving as a waste liquid reservoir exists, that region is also excluded.

Both the first bonding treatment and the second bonding treatment may be treatments with an adhesive, or the first bonding treatment may be a treatment with an adhesive and the second bonding treatment may be a welding treatment.

In the former case, in order to change the adhesive strength of the adhesive between the bonded part of the end face of the wall part of the substrate and the film, and the other bonded areas, it is preferable to perform a surface treatment on either the end face of the wall part of the substrate or the bonding region other than the end face of the wall part. By performing a surface treatment and then applying the adhesive, the bonding strength of the adhesive can be changed.

As the adhesive, it is preferable to use an adhesive having a gel fraction after curing of 60% or more, and more preferable to use an adhesive of 80% or more. By using an adhesive having a gel fraction after curing of 60% or more, pressure can be applied into the flow path sufficient to penetrate the wall part 114. On the other hand, with an adhesive having a gel fraction after curing of less than 60%, the degree of crosslinking is insufficient, resulting in a structure that allows air to pass through. Therefore, the pressure is lost before the pressure inside the flow path reaches 30 to 300 kPa, which is necessary to penetrate the wall part 114, making it impossible to penetrate the wall part 114.

The gel fraction after curing of the adhesive can be measured, for example, by the following method.

An adhesive is applied onto a substrate to prepare a test piece of a certain area. The test piece is immersed in toluene at 23°C for 24 hours, then removed from the toluene and dried under conditions of 110°C for 1 hour. The weight of the test piece after drying is measured, and the gel fraction is calculated using the following formula (1).

$$\text{Gel fraction (wt\%)} = 100 \times (W2 - W0) / (W1 - W0) \quad (1)$$

(WO: weight of substrate, W1: weight of test piece before immersion, W2: weight of test piece after immersion and drying)

Examples of adhesives having a gel fraction after curing of 60% or more include, although depending on the composition and curing conditions, (meth)acrylic resin-based adhesives, natural rubber adhesives, urethane resin-based adhesives, ethylene-vinyl acetate resin emulsion adhesives, ethylene-vinyl acetate resin-based adhesives, epoxy resin-based adhesives, vinyl chloride resin solvent-based adhesives, chloroprene rubber-based adhesives, cyanoacrylate-based adhesives, silicone-based adhesives, styrene-butadiene rubber solvent-based adhesives, nitrile rubber-based adhesives, nitrocellulose-based adhesives, phenol resin-based adhesives, modified silicone-based adhesives, polyester-based adhesives, polyamide-based adhesives, polyimide-based adhesives, olefin resin-based adhesives, vinyl acetate resin emulsion-based adhesives, polystyrene resin solvent-based adhesives, polyvinyl alcohol-based adhesives, polyvinylpyrrolidone resin-based adhesives, polyvinyl butyral-based adhesives, polybenzimidazole adhesives, polymethacrylate resin solvent-based adhesives, melamine resin-based adhesives, urea resin-based adhesives, resorcinol-based adhesives, and the like. Adhesives having a gel fraction after curing of 60% or more can be used alone or as a mixture of two or more types.

The adhesive having a gel fraction after curing of 60% or more is preferably a photocurable type (either radical-reactive or cationically polymerizable), and more preferably an ultraviolet-curable type (UV-curable type). With a UV-curable adhesive, the curing reaction can be started quickly and bonding can be achieved by irradiating with UV after the application process. As the UV-curable adhesive, acrylic-based UV-curable adhesives such as UVX-8204 (manufactured by Denka Company Limited), UVX-8400 (Denka Company Limited), SX-UV100A (manufactured by Cemedine Co., Ltd.), SX-UV200 (Cemedine Co., Ltd.), BBX-UV300 (Cemedine Co., Ltd.), U-1340 (Chemitech Inc.), U-1455B (Chemitech Inc.), U-1558B (Chemitech Inc.), Aronix UV-3000 (Toagosei Co., Ltd.), TB3094 (ThreeBond Co., Ltd.), and Hitaloid 7975D (Hitachi Chemical Company, Ltd.) are more preferable. Acrylic-based UV-curable adhesives exhibit good adhesion to a wide range of plastic materials and can achieve rapid strength development after UV irradiation. The viscosity of the adhesive used to bond the film 120 onto the substrate 110 is, for example, preferably 2,000 to 31,000 mPa·s.

When applying the adhesive to the end face of the wall part, half or more of the end face of the wall part (the first half of the end face in the longitudinal direction of the flow path) may be left uncoated with adhesive, such that pressure is applied from the uncoated side. That is, on the end face of the wall part, the adhesive may be applied only to a region of the end face covering 1/2 or less of the end face on the downstream side of the flow path (i.e., the latter half of the end face in the longitudinal (flow) direction of the flow path). "Half (1/2) or less" is, for example, 1/2 to 1/5. That is, the end face of the wall part of the substrate may be bonded to the film with the adhesive applied to a region of the end face covering 1/2 or less of the end face on the downstream side of the flow path. In this embodiment, when pressure is applied from the upstream side, the peel angle between the end face and the film increases, and the peel stress can be made smaller than when the peel angle is 0°.

In the embodiment where the first bonding treatment is a treatment with an adhesive and the second bonding treatment is a welding treatment, the bonded portion between the end face of the wall part of the substrate and the film is bonded with an adhesive having a gel fraction after curing of 60% or more, and the other bonded areas are joined by welding.

Examples of welding include heat sealing (also called thermal welding), laser welding, ultrasonic welding, and the like, and it is particularly preferable to use heat sealing. With heat sealing, efficient welding can be achieved with a simple apparatus consisting of a pressing mechanism and a heater.

To accurately heat seal only the desired bonding region of the substrate 110 other than the end face of the wall part, it is preferable to use a stainless steel or aluminum jig having a convex shape identical to the desired bonding region. The film 120 is placed on the convex shape of the jig, and the substrate 110 is placed on top of it such that the bonding surface 115 faces the film 120 side. By performing a high-temperature heat treatment with a temperature-controllable press machine, bonding of only the convex-shaped region can be achieved. The pressing direction is not particularly limited and may be from the film side, the substrate side, or both sides.

The pressure depends on the materials to be bonded and is not particularly limited as long as bonding is achieved and the flow path shape is not deformed, but it is preferably 0.1 to 12.0 MPa.

The bonding temperature depends on the materials to be bonded, but it can be set to ±10°C of the glass transition temperature of the substrate 110 and the film 120. More preferably, the surface of the substrate 110 can be set 1 to 10°C lower than the glass transition temperature of the substrate 110, and the surface of the film 120 can be set 1 to 10°C higher than the glass transition temperature of the film 120. This makes it possible to perform heat sealing while maintaining the flow path shape of the substrate 110. Bonding can also be achieved by setting the surface of the substrate 110 higher than the glass transition temperature, but this is not practical because the flow path shape deforms.

The time required for heat sealing depends on the materials to be bonded and is not particularly limited as long as bonding is achieved and the flow path shape is not deformed, but it is, for example, preferably 5 seconds to 2 hours.

Laser welding enables stronger and higher-precision bonding than heat sealing, so it is preferably used when strength is required for the bonded areas other than the end face of the wall part. Good bonding can be achieved with highly transparent amorphous resins such as COP, polycarbonate, and PMMA, but the material is not particularly limited. The transparency of one resin required for laser welding is, for example, a laser transmittance of 30% or more. The other resin can be a gray or black material with high light absorption, and high light absorption markings can also be applied only to the laser-welded part of the transparent material.

Laser welding of the substrate 110 and the film 120 can be achieved by placing the substrate 110 with a laser transmittance of 30% or more on the upper side, placing the film 120 that absorbs the laser on the lower side so that it aligns with the substrate 110, and irradiating the laser from the upper side. When using a substrate 110 that absorbs laser and a film 120 with a laser transmittance of 30% or more, the laser may be irradiated from the lower side, or the substrate 110 may be placed on the lower side and the film 120 on the upper side and the laser irradiated from the upper side, which can be changed as appropriate.

The laser method is not particularly limited, but it is particularly preferable to use the heat-sink-type laser resin welding method. With the heat-sink type, the substrate 110 and the film 120 can be bonded even if they are of the same transparent material, and the heat sink (heat radiator) prevents temperature rise on the member surface and inside, allowing a high-precision flow path to be obtained.

Ultrasonic welding can bond thermoplastic resins even if they are colored or dirty, and allows simultaneous bonding of multiple points in a short time. The ultrasonic treatment time is not particularly limited as long as bonding is achieved, but it is, for example, preferably 1 to 10 seconds. The operating frequency of the ultrasonic welding machine is preferably 15 kHz to 200 kHz.

The end face of the wall part of the substrate is bonded to the film with an adhesive having a gel fraction after curing of 60% or more, so the bonded part peels at a pressure of 200 kPa or more and less than 300 kPa. On the other hand, the other bonded areas of the substrate are joined by welding technology such as heat sealing, so, although it depends on the flow path shape, a pressure of about 1000 kPa or more is required for peeling, and they do not peel at a pressure of 200 kPa or more and less than 300 kPa. Therefore, the bonded portion between the end face of the wall part of the substrate and the film can be selectively peeled by a pressure of 200 kPa or more and less than 300 kPa.

Next, an embodiment will be described in which the bonding strength is changed by performing a surface treatment on either the end face of the wall part of the substrate or the bonding region other than the end face of the wall part, and then applying an adhesive.

In one embodiment, the end face of the wall part of the substrate is not surface-treated, and the bonding region of the substrate other than the end face of the wall part is surface-treated so that the bonding surface of the substrate has a water contact angle of 10° to 40°, and then bonded with an adhesive having a gel fraction after curing of 60% or more. Although it depends on the type of adhesive used, the water contact angle of the bonding surface of the substrate is preferably 10° to 40°. By setting the water contact angle of the bonding surface of the substrate to 10° to 40°, the wettability of the substrate to the adhesive is improved, allowing the adhesive to conform to the fine irregularities (10 nm or less) on the substrate surface, and the adhesive solidifies while wet on the substrate surface, thereby exhibiting a strong bonding force. Even if the water contact angle of the substrate is less than 10°, strong bonding can be expected, but due to high wettability, it becomes difficult to precisely apply the adhesive only to a predetermined location, increasing the risk of the adhesive flowing into the flow path, making it unsuitable for practical use. On the other hand, if the water contact angle exceeds 40°, the adhesive does not wet the bonding surface of the substrate uniformly, and a sufficient improvement in bonding strength cannot be expected.

Here, as the hydrophilic treatment to make the water contact angle 10° to 40°, atmospheric pressure plasma treatment, vacuum plasma treatment, corona treatment, excimer treatment, or the like can be used, but vacuum plasma is preferable because it can be processed under room temperature conditions and there is no concern about deformation of the flow path shape. For the vacuum plasma treatment gas, oxygen, nitrogen, argon, or the like can be used, but it is particularly preferable to use air. Specifically, plasma made of general air containing about 78% nitrogen, 21% oxygen, 1% argon by volume, and even lower proportions of carbon dioxide, etc., is used under a medium vacuum of 100 to 0.1 Pa.

The end face of the wall part of the substrate is bonded to the film with an adhesive having a gel fraction after curing of 60% or more without surface treatment (the water contact angle of the substrate surface without surface treatment is usually 80° to 100° depending on the type of substrate), so the bonded part peels at a pressure of 200 kPa or more and less than 300 kPa. On the other hand, the other bonded areas of the substrate are surface-treated so that the bonding surface of the substrate has a water contact angle of 10° to 40° and then bonded with an adhesive having a gel fraction after curing of 60% or more, so a pressure of about 500 kPa is required for peeling, and they do not peel at a pressure of 200 kPa or more and less than 300 kPa. Therefore, the bonded portion between the end face of the wall part of the substrate and the film can be selectively peeled by a pressure of 100 kPa or more and less than 200 kPa.

In another embodiment, the bonded portion between the end face of the wall part of the substrate and the film is bonded with an adhesive having a gel fraction after curing of 60% or more after the end face of the wall part has been surface-treated to have a water contact angle of 110° or more, and the other bonded areas are bonded with an adhesive having a gel fraction after curing of 60% or more.

By setting the water contact angle of the bonding surface of the substrate 110 to 110° or more, the wettability of the substrate to the adhesive decreases, and the bonding strength decreases. Examples of the surface treatment (hydrophobic treatment) to make the water contact angle 110° or more include fluorine coating or silicone treatment. Examples of fluorine coating agents include Fluorosurf FS-1610C-4.0 (manufactured by FluoroTechnology Co., Ltd.), water/oil repellent fluorine coating agent GF03 (Gast Japan Co., Ltd.), Guard Surf AZ-6000 (Harves Co., Ltd.), and fluorine coating agent INT series (Noda Screen Co., Ltd.). In that case, a fluorine-based resin concentration of 0.2 to 0.8% is preferable. Examples of silicone treatment agents include dimethyl silicone, methylphenyl silicone, methyl hydrogen silicone, and modified silicones in which various organic groups are introduced into some of the methyl groups. In that case, a spray type with a silicone concentration of 0.01 to 10% is preferable.

The end face of the wall part of the substrate is surface-treated to have a water contact angle of 110° or more and then bonded to the film with an adhesive having a gel fraction after curing of 60% or more, so the bonded part peels at a pressure of 80 to 150 kPa. On the other hand, the other bonded areas of the substrate are bonded with an adhesive having a gel fraction after curing of 60% or more without surface treatment (the water contact angle of the substrate surface without surface treatment is usually 80° to 100°), so a pressure of about 200 to 300 kPa is required for peeling, and they do not peel at a pressure of 80 to 150 kPa. Therefore, the bonded portion between the end face of the wall part of the substrate and the film can be selectively peeled by a pressure of 80 to 150 kPa.

With reference to FIG. 2, the adhesive application process in the embodiment where only the end face of the wall part is fluorine-coated and then the substrate and film are bonded with an adhesive having a gel fraction after curing of 60% or more will be described. However, the adhesive application process can be similarly performed in the embodiment where the bonding region other than the end face of the wall part is plasma-treated and bonded with an adhesive having a gel fraction of 60% or more. First, a substrate 110 having two through-holes serving as an inlet and an outlet, a groove serving as a flow path, a depression serving as a liquid reservoir, and a wall part on its surface is prepared, and the end face of the wall part is fluorine-coated. The fluorine coating can be performed, for example, using Fluorosurf FS-1610C-4.0. Next, an adhesive 117 is applied to the bonding surface 115 by screen printing. FIG. 2 is a diagram showing the substrate 110 of the microchip 100 after applying the adhesive.

In another embodiment, the bonded part between the wall part and the film is bonded with an adhesive having a gel fraction after curing of 60% or more applied only in a region of the end face covering 1/2 or less of the end face on the downstream side of the flow path, after the end face has been surface-treated to have a water contact angle of 110° or more, or after the end face of the wall part of the substrate is not surface-treated and the bonding region of the substrate other than the end face of the wall part is surface-treated to have a water contact angle of 10° to 40°, and the other bonded areas are bonded with an adhesive having a gel fraction after curing of 60% or more.

Thereby, the bonded portion between the end face of the wall part of the substrate and the film can be selectively peeled by a pressure of 30 to 80 kPa. On the other hand, the other bonded areas of the substrate are bonded with an adhesive having a gel fraction after curing of 60% or more without surface treatment, so a pressure of about 200 to 300 kPa is required for peeling, and they do not peel at a pressure of 30 to 80 kPa. Therefore, the bonded portion between the end face of the wall part of the substrate and the film can be selectively peeled by a pressure of 30 to 80 kPa.

The adhesive 117 is applied to the region of the bonding surface 115 of the substrate 110 excluding the groove 111a, the liquid reservoir 116, the inlet 112, and the outlet 113.

To apply it accurately, the adhesive 117 is preferably applied by a printing technique, and preferably by screen printing. By using screen printing, even when the screen printing plate corresponding to the entire area of the substrate 110 is filled with the adhesive 117, the adhesive 117 is transferred to the application region in contact with the screen printing plate, but the adhesive 117 is not transferred to the groove 111a and the depression serving as the liquid reservoir 116, which are not in contact. Therefore, the adhesive 117 can be accurately applied to the intended application region.

The thickness of the adhesive 117 applied to the substrate 110 is preferably 5 to 15 μm. To control the thickness of the adhesive 117, the mesh count per inch of the screen is, for example, preferably 500 to 730. The opening ratio of the mesh is, for example, preferably 39 to 47%. The thickness of the mesh is, for example, preferably 15 to 28 μm. Thereby, the thickness of the applied adhesive 117 is preferably 5 to 15 μm.

As other application methods of the adhesive 117 to the substrate 110, the adhesive can be precisely applied to the intended application region by inkjet printing, gravure printing, a dispenser, or the like. In these application techniques, if the adhesive 117 is ejected into the groove 111a, the adhesive 117 applied in the groove 111a will change the shape of the flow path 111. Therefore, the printing process or the operation of the dispenser should be controlled by a program so that the adhesive 117 is applied to the intended application region, by capturing the position of the groove 111a of the substrate 110 with an image, or by fixing the positions of the printing stage and the substrate 110.

<Bonding Step>

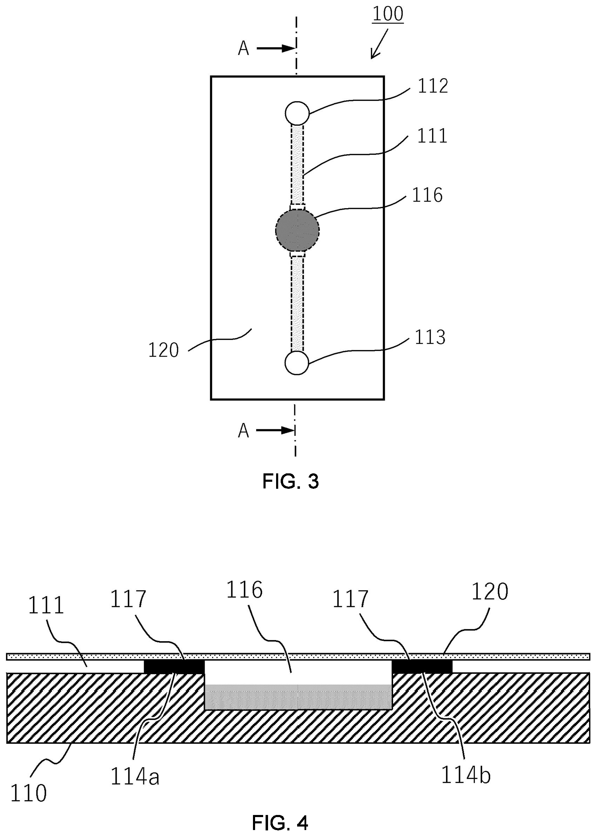

With reference to FIG. 3, a step of preparing a film 120, storing liquid in the liquid reservoir, and then bonding the film 120 to the substrate 110 will be described. FIG. 3 is a completed view of the microchip with the film 120 bonded to the substrate 110. FIG. 3 shows a plan view of the microchip 100 obtained by bonding the bonding surface 115, on which the groove 111a and the liquid reservoir 116 of the substrate 110 are carved, and the film 120. The flow path 111 formed inside the microchip 100 is indicated by a dashed line.

By laminating and bonding the film 120 onto the substrate 110, the upper parts of the groove 111a and the depression serving as the liquid reservoir 116 are covered with the film 120, forming the flow path 111 through which the liquid passes, and the liquid reservoir 116. Also, the liquid is retained within the liquid reservoir 116 by the wall parts 114a, 114b. The adhesive 117 is cured by UV irradiation, forming a bonded part. In this way, by applying the adhesive to a predetermined application region by screen printing and laminating and bonding the film 120 onto the substrate 110, the bonding of the substrate 110 and the film 120 and the formation of a structure that blocks the outflow of liquid can be realized in the same step.

Further, the through-holes of the substrate 110 are sealed on the bonding surface 115 side by laminating the film 120, and only the surface opposite to the bonding surface 115 serves as the inlet. Thereby, the through-holes of the substrate 110 function as the inlet 112 and the outlet 113.

Hereinafter, the passage of the liquid stored in the liquid reservoir 116 will be described with reference to FIGS. 3 to 8. FIG. 4 is a diagram showing a part of the A-A cross-section of the microchip 100 shown in FIG. 3.

For example, a solution of a reagent that reacts with a target substance in a liquid sample is stored in the liquid reservoir 116 (FIGS. 3 and 4). The reagent that reacts with the target substance in the liquid sample may be stored in the liquid reservoir after being supported on a carrier such as beads. Also, a stirrer for stirring the reagent that reacts with the target substance in the liquid sample or the carrier such as beads supporting it may be stored in the liquid reservoir.

The substrate 110 and the film 120 are bonded, and the end faces 114a, 114b of the wall part and the film 120 are sealed by being bonded with an adhesive or the like, so the liquid in the liquid reservoir 116 does not flow out into the flow path 111.

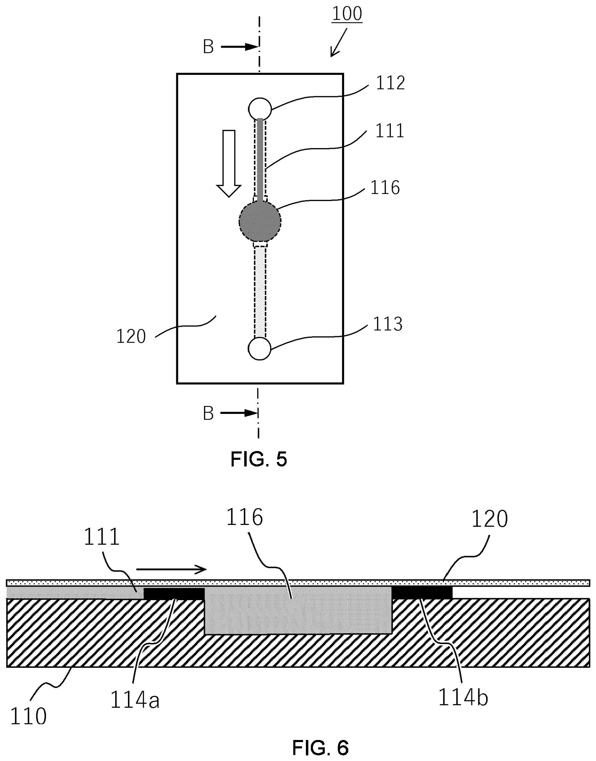

Then, at the time of use, by introducing the liquid sample under pressure from the inlet 112 with a syringe or the like, the liquid sample passes through the flow path and reaches the wall part before the liquid reservoir. Here, the end face 114a of the wall part 114 and the film peel apart due to the pressure, and the liquid sample is passed into the liquid reservoir and mixed with the reaction solution stored in advance in the liquid reservoir (FIGS. 5 and 6).

Among the bonded areas of the substrate and the film, those other than the bonded part between the end face of the wall part and the film are bonded with a bonding strength stronger than the bonding strength at the bonded part between the end face of the wall part and the film, so they do not peel even with the above pressure, allowing stable control of liquid passage.

FIG. 6 is a diagram showing a part of the B-B cross-section of the microchip 100 shown in the diagram where the end face 114a of the wall part 114 and the film 120 have peeled, and the liquid sample has passed through the flow path 111 into the liquid reservoir 116.

A substance in the liquid sample and a reactive substance (reagent) in the reaction solution react, and by detecting this reaction, the liquid sample can be analyzed. The target substance in the sample can be measured by observing or detecting the reaction. Examples of the reaction include, but are not limited to, color reactions, luminescence reactions, amplification reactions, and agglutination reactions.

Examples of the liquid sample include liquid samples obtained from living bodies such as blood and urine or their dilutions, extracts from living bodies such as plants and animals, naturally occurring water such as rivers, seas, and rain, washing liquids, waste liquids, and the like. The components (target substances for measurement) in the sample are also not particularly limited, and examples include proteins, nucleic acids, low-molecular-weight compounds, sugars, and the like.

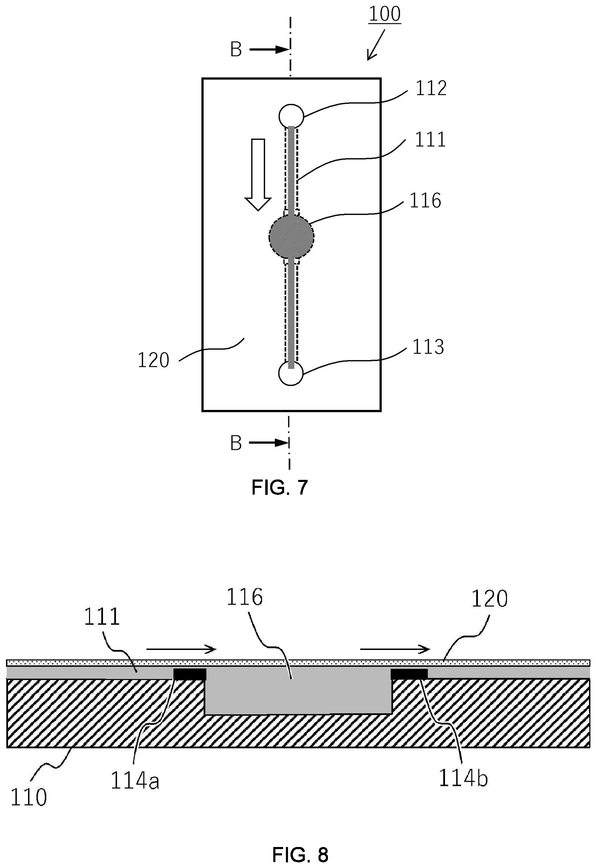

After the reaction, by further applying pressure for liquid passage, the downstream wall part 114b separating the liquid reservoir and the downstream flow path can be peeled, allowing the liquid (mixed liquid) in the liquid reservoir to flow out into the downstream flow path (FIGS. 7 and 8). FIG. 8 is a diagram showing a part of the A-A cross-section of the microchip 100 shown in FIG. 7. By further introducing liquid under pressure from the inlet 112, the liquid in the liquid reservoir 116 peels the end face 114b and the film 120, passes through the gap between the end face 114b and the film 120, and flows into the downstream side of the flow path 111. The liquid that has flowed out can be discharged from the outlet 113 after passing through the downstream flow path. A waste liquid reservoir for storing the discharged liquid may be provided in a part of the downstream flow path.

If it is not necessary to discharge the liquid from the liquid reservoir, the downstream flow path and the outlet do not need to be provided.

<Examples>

Hereinafter, the present invention will be specifically described with reference to Examples, but the present invention is not limited to the following embodiments.

Example 1

<Production of Microchip 1>

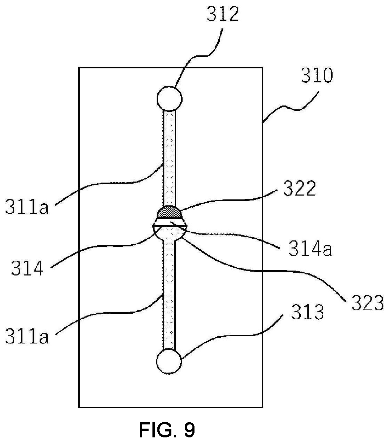

A substrate 310 (milled product made of COP resin) shown in FIG. 9 (size 59.4 × 26.2 mm, thickness 3.0 mm) was prepared. In the substrate 310, the length of the groove 311a forming the flow path was 47.4 mm, the depth was 500 μm, the width at the inlet part was 500 μm, and it had a reservoir 322 with a semi-circular shape (upper half) obtained by cutting a 4 mm diameter circle in half, centered at a point 37.55 mm from the inlet in the direction of the outlet. The side receiving the liquid passed from the reservoir 322 was a reservoir 323 with a semi-circular shape (lower half) obtained by cutting a 4.5 mm diameter circle in half. A wall part 314 with a width of 2 mm was provided between the flow path receiving from the reservoir 322 (between the reservoir 322 and the reservoir 323). In the substrate, the holes serving as the inlet 312 and the outlet 313 were through-holes with a circular cross-section and an inner diameter of 2 mm.

As the substrate 310 and the ZF14-100 film (Zeon Corporation: made of COP) used for bonding, untreated ones and ones surface-treated with vacuum plasma were prepared. Regarding the vacuum plasma surface treatment for the substrate 310, ones treated on the flat parts other than the wall part 314 and ones treated on the entire surface including the wall part 314 were prepared. When not irradiating the wall part 314 with vacuum plasma, the wall part 314 was masked with stainless steel tape. The vacuum plasma conditions were as follows: under a medium vacuum of 11 Pa, using general air containing about 78% nitrogen, 21% oxygen, 1% argon by volume, and even lower proportions of carbon dioxide, etc., the voltage supplied to the plasma power source was 10 V, and irradiation was performed for 10 seconds. As a result, the water contact angle of the treated surface became 25°. The water contact angle of the substrate surface without surface treatment was 85°.

The bonding of the substrate 310 and the film was performed using an adhesive. As the adhesive, TB3094, a solvent-free radical-reactive acrylic UV-curable adhesive with a gel fraction of 90% or more, and a uniquely formulated solvent-free radical-reactive acrylic UV-curable adhesive with a gel fraction of 50% were used. The adhesive was applied to the surface of the substrate 310 where the groove 311a was provided, by screen printing. In the screen plate used, the mesh count was 640, the opening ratio was 39%, and the applied thickness of the adhesive was about 7 $\mu$m.

The adhesive-applied surface of the substrate 310 was laminated with the film, and a UV-LED light source was used to irradiate ultraviolet rays with a wavelength of 365 nm for 10-20 seconds, thereby starting the curing reaction of the adhesive and bonding the film onto the substrate 310.

<Evaluation of Microchip 1>

It was evaluated whether selective peeling of the wall part 314 and the film can be achieved by selectively increasing and peeling the bonding strength of the locations of the substrate 310 other than the wall part 314. In addition, the gel fraction after curing of the adhesive that can maintain sufficient pressure within the flow path to penetrate the wall part 314 was confirmed.

The inlet 312 was connected to a pressurizing syringe pump using a connector made of a silicone tube and an ABS resin bush. A pressure sensor was assembled to the syringe pump to record the pressure at the moment the adhesive on the end face 314a of the wall part 314 peeled, using a peak hold function. As a result of pressurizing with the syringe pump, in the microchip surface-treated on the locations other than the wall part 314 and bonded with TB3094 having a gel fraction of 90% or more, the adhesive on the end face 314a of the wall part 314 peeled at 250 kPa, and the distilled water subsequently introduced flowed to the flow path ahead. On the other hand, in the microchip uniformly surface-treated on the entire surface including the wall part 314 and bonded with TB3094 having a gel fraction of 90% or more, liquid leakage occurred around the wall part 314 as a starting point at 240 kPa.

From this result, it was found that by making the adhesive strength of the locations other than the wall part 314 greater than the adhesive strength of the bonded part of the wall part 314 through surface treatment, selective peeling of the wall part 314 can be achieved.

With the uniquely formulated adhesive with a gel fraction of 50%, regardless of the presence or absence of surface treatment on the wall part 314, air leaked from the adhesive layer before reaching the pressure necessary to penetrate the wall part 314, and it was not possible to peel the end face 314a of the wall part 314.

From this result, it was found that an adhesive with a low gel fraction after curing and a porous-like structure is not suitable for use in peeling the wall part 314.

Example 2

It was evaluated whether selective peeling of the wall part 314 and the film can be achieved by selectively reducing only the bonding strength of the end face 314a of the wall part 314 of the substrate 310.

<Production of Microchip 2>

The substrate 310 used for production was the same as in Example 1. The film used was ZF14-100. The adhesive used was TB3094 with a gel fraction of 90% or more.

The end face 314a of the wall part 314 of the substrate 310 before bonding was pretreated with a fluorine coating agent or a silicone coating agent. As the fluorine coating agent, Fluorosurf FS-1610C-4.0 (manufactured by Fluoro-Technology Co., Ltd.) was applied, and as the silicone coating agent, KF96SP (manufactured by Shin-Etsu Chemical Co., Ltd.) was applied, and each was dried. By these treatments, the water contact angle of the end face 314a became 110° or more. Otherwise, a microchip was produced in the same manner as in Example 1.

<Evaluation of Microchip 2>

The inlet 312 was connected using a connector made of a silicone tube and an ABS resin bush, and the pressure required to peel the end face 314a and the adhesive was measured in the same manner as in Example 1. Pressure was applied from the through-hole on the reservoir 322 side. As a result of the experiment, in the microchip bonded after applying the fluorine coating agent, the adhesive on the end face 314a of the wall part 314 peeled at 130 kPa. In the microchip bonded after applying the silicone coating agent, the adhesive on the end face 314a of the wall part 314 peeled at 125 kPa. In both microchips, the distilled water subsequently introduced flowed to the flow path ahead. From these results, it was found that by reducing the adhesive strength of the end face 314a of the wall part 314, selective peeling of the wall part 314 can be achieved.

Example 3

<Production of Microchip 3>

As an example of the embodiment of selectively reducing only the bonding strength of the end face 314a of the wall part 314 of the substrate 310, it was evaluated whether the pressure required for selective peeling of the wall part 314 and the film could be reduced by applying the adhesive only to the region of the end face 314a of the wall part covering 1/2 or less of the end face on the downstream side (reservoir 323 side) of the flow path.
The substrate 310 used for production was the same as in Example 1. The film used was ZF14-100. The adhesive used was TB3094 with a gel fraction of 90% or more.
The end face 314a of the wall part 314 of the substrate 310 before bonding was coated with the fluorine coating agent Fluorosurf FS-1610C-4.0 (manufactured by FluoroTechnology Co., Ltd.) and dried. By these treatments, the water contact angle of the end face 314a became 110° or more.
The adhesive TB3094 was applied to the surface of the substrate 310 where the groove 311a was provided, by screen printing. At this time, by providing a screen mask, a region of about half on the upstream side (reservoir 322 side) of the end face of the wall part 314 was left uncoated with adhesive (adhesive was applied to a region covering about 1/2 of the end face on the downstream side (reservoir 323 side).

<Evaluation of Microchip 3>

The inlet 312 was connected using a connector made of a silicone tube and an ABS resin bush, and the pressure required to peel the end face 314a and the adhesive was measured in the same manner as in Example 1. Pressure was applied from the through-hole on the reservoir 322 side. As a result of the experiment, in the microchip bonded with the adhesive only on half of the end face 314a after applying the fluorine coating agent, the adhesive on the end face 314a of the wall part 314 peeled at 30 kPa, and the distilled water subsequently introduced flowed to the flow path ahead. In this way, by selectively reducing only the bonding strength of the end face 314a of the wall part 314 of the substrate 310 and applying the adhesive only to the region of the end face 314a covering 1/2 or less of the end face on the downstream side (reservoir 323 side) of the flow path, the pressure required for selective peeling of the wall part 314 and the film could be reduced to 30 kPa.

Reference Signs List

100... microchip, 110... substrate, 111... flow path, 111a... groove, 112... inlet, 113... outlet, 114a, 114b... wall part, 115... bonding surface, 116... liquid reservoir, 117... adhesive, 120... film, 310... substrate, 311a... groove, 312... inlet, 313... outlet, 314... wall part, 314a, 314b, 314c... end face, 317... adhesive, 320... film, 322... semi-circular reservoir (diameter 4mm), 323... semi-circular reservoir (diameter 4.5mm)

**Claims**

1. A microchip for liquid sample analysis having a flow path and a liquid reservoir therein, comprising:

    a substrate having a bonding surface provided with a groove forming the flow path, a depression provided in a part of the groove and serving as the liquid reservoir storing a liquid, and a wall part provided between the groove and the depression to block outflow of the liquid from the liquid reservoir to the flow path; and
    a film bonded to the bonding surface of the substrate, covering the groove to form the flow path and the liquid reservoir, wherein
    among the bonded areas of the substrate and the film, the bonded portion between the end face of the wall part of the substrate and the film has a first bonding strength such that it peels off upon application of pressure of 30 kPa or more and less than 300 kPa, and the other bonded areas have a bonding strength stronger than the first bonding strength.

2. The microchip according to claim 1, wherein

the bonded portion between the end face of the wall part of the substrate and the film has been bonded with an adhesive having a gel fraction after curing of 60% or more, and

for the other bonded areas of the substrate and the film, the bonding surface of the substrate has been surface-treated to have a water contact angle of 10° to 40° and bonded to the film with an adhesive having a gel fraction after curing of 60% or more.

3. The microchip according to claim 2, wherein the end face of the wall part of the substrate has been bonded to the film with the adhesive applied to a region of the end face covering 1/2 or less of the end face on the downstream side of the flow path.

4. The microchip according to claim 2, wherein the surface treatment is atmospheric pressure plasma treatment, vacuum plasma treatment, corona treatment, or excimer treatment.

5. The microchip according to claim 1, wherein

the bonded portion between the end face of the wall part of the substrate and the film has been bonded with an adhesive having a gel fraction after curing of 60% or more, and

the other bonded areas have been joined by welding.

6. The microchip according to claim 5, wherein the welding is heat sealing, laser welding, or ultrasonic welding.

7. The microchip according to claim 1, wherein the end face of the wall part has been surface-treated to have a water contact angle of 110° or more and the bonded portion between the end face of the wall part of the substrate and the film has been bonded with an adhesive having a gel fraction after curing of 60% or more, and the other bonded areas have been bonded with an adhesive having a gel fraction after curing of 60% or more.

8. The microchip according to claim 7, wherein the end face of the wall part of the substrate has been bonded to the film with the adhesive applied to a region of the end face covering 1/2 or less of the end face on the downstream side of the flow path.

9. The microchip according to claim 7, wherein the surface treatment is fluorine coating or silicone coating.

10. The microchip according to any one of claims 1 to 9, wherein the substrate or the film has through-holes serving as an inlet for pressurizing the liquid and an outlet for the liquid or air to flow out, at positions corresponding to both ends of the flow path formed by the substrate and the film.

11. The microchip according to any one of claims 1 to 9, wherein the liquid stored in the liquid reservoir is pressurized via the inlet using a microsyringe or a pump, or by pressing with a finger.

12. The microchip according to any one of claims 1 to 9, wherein the substrate is made of any of plastic, silicone, and glass.

13. The microchip according to any one of claims 1 to 9, wherein the film has a Young's modulus (tensile elastic modulus) of, for example, 50 to 2800 MPa.

14. The microchip according to claim 13, wherein the film is made of cycloolefin polymer (COP), cycloolefin copolymer (COC), polymethyl methacrylate (PMMA), polystyrene (PS), polycarbonate (PC), or polyethylene terephthalate (PET).

FIG. 1

FIG. 2

FIG. 3

FIG. 4

FIG. 5

FIG. 6

EP 4 764 506 A1

FIG. 7

FIG. 8

18

FIG. 9

## INTERNATIONAL SEARCH REPORT

| | International application No. |
|---|---|
| | **PCT/JP2024/029002** |

**A. CLASSIFICATION OF SUBJECT MATTER**

*G01N 35/08*(2006.01)i; *B01J 19/00*(2006.01)i; *B81B 1/00*(2006.01)i; *B81C 1/00*(2006.01)i; *G01N 37/00*(2006.01)i
FI: G01N35/08 A; G01N37/00 101; B01J19/00 321; B81B1/00; B81C1/00

According to International Patent Classification (IPC) or to both national classification and IPC

**B. FIELDS SEARCHED**

Minimum documentation searched (classification system followed by classification symbols)

G01N35/08; B01J19/00; B81B1/00; B81C1/00; G01N37/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2012-517595 A (NORTHWESTERN UNIVERSITY) 02 August 2012 (2012-08-02) entire text, all drawings | 1-14 |
| A | JP 2007-162899 A (PENTAX CORP.) 28 June 2007 (2007-06-28) entire text, all drawings | 1-14 |
| A | JP 2011-524313 A (BOEHRINGER INGELHEIM MICROPARTS GMBH) 01 September 2011 (2011-09-01) entire text, all drawings | 1-14 |
| A | JP 2019-113438 A (SEKISUI CHEMICAL CO., LTD.) 11 July 2019 (2019-07-11) entire text, all drawings | 1-14 |

☐ Further documents are listed in the continuation of Box C.    ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **09 October 2024** | **22 October 2024** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

**INTERNATIONAL SEARCH REPORT**
Information on patent family members

| International application No. |
| --- |
| **PCT/JP2024/029002** |

| Patent document cited in search report | | | Publication date (day/month/year) | Patent family member(s) | | | Publication date (day/month/year) |
| --- | --- | --- | --- | --- | --- | --- | --- |
| JP | 2012-517595 | A | 02 August 2012 | US | 2012/0107811 | A1 | |
| | | | | entire text, all drawings | | | |
| | | | | CA | 2751654 | A1 | |
| | | | | entire text, all drawings | | | |
| | | | | CN | 102387966 | A | |
| | | | | entire text, all drawings | | | |
| JP | 2007-162899 | A | 28 June 2007 | (Family: none) | | | |
| JP | 2011-524313 | A | 01 September 2011 | US | 2011/0186466 | A1 | |
| | | | | entire text, all drawings | | | |
| | | | | WO | 2009/152952 | A1 | |
| | | | | entire text, all drawings | | | |
| | | | | CN | 102105227 | A | |
| | | | | entire text, all drawings | | | |
| JP | 2019-113438 | A | 11 July 2019 | (Family: none) | | | |

Form PCT/ISA/210 (patent family annex) (July 2022)

**EP 4 764 506 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009168667 A